# EUROPEAN PATENT SPECIFICATION

(11) **EP 3 763 337 B1**
(45) Date of publication and mention of the grant of the patent: **07.05.2025**
(21) Application number: 19184899.3
(22) Date of filing: 08.07.2019
(51) Int. Cl.: A61F 2/46, A61B 17/17, A61F 2/30

(54) **MANDREL COMPRISING POSITIONING MARK**
DORN MIT POSITIONIERUNGSMARKIERUNG
MANDRIN COMPRENANT UNE MARQUE DE POSITIONNEMENT

(43) Date of publication of application: 13.01.2021
(73) Proprietor: Episurf IP-Management AB, 114 49 Stockholm (SE)
(72) Inventor: SPÅNGBERG, Jeanette, 79392 Leksand (SE); JULIN, Johan, 125 70 Älvsjö (SE)
(74) Representative: Rouse AB

(56) References cited:
- WO-A1-2016/161026
- US-A1- 2013 131 741
- US-A1- 2014 249 781
- US-A1- 2016 199 198
- US-A1- 2019 038 430

## Description

### Field of the invention

This present invention relates generally to the field of designing tools for use during replacement of damaged cartilage in an articulate surface in a joint. The present invention also relates to tools such as mandrels for rotationally positioning and inserting a medical implant in a recess made in a joint using a mandrel for hammering, pressing and/or pushing the implant into the recess. The closest prior art is document US 2016/199198 A1, which defines the preamble of claim 1.

### Background

Pain and overuse disorders of the joints in the body is a common problem. The weight-bearing and articulate surfaces of the knees and other joints are covered with a layer of soft tissue that typically comprises a significant amount of hyaline cartilage. The friction between the cartilage and the surrounding parts of the joint is very low, which facilitates movement of the joints under high pressure. The cartilage is however prone to damage due to disease, injury or chronic wear. Moreover, it does not readily heal after damages, as opposed to other connective tissue, and if healed the durable hyaline cartilage is often replaced by less durable fibrocartilage. This means that damages of the cartilage gradually become worse. Along with injury/disease comes a problem with pain which results in handicap and loss of function. It is therefore important to have efficient means and methods for repairing damaged cartilage in knee joints.

The advantages of implants have stimulated a further development of smaller implants that can be implanted with less invasive surgery. In this development there has also been an effort to achieve small joint implants, suitable for repair of small cartilage injuries that have a minimal influence on the surrounding parts of the joint. In the surgical operation of implanting such small implants, it is critical that the implant is positioned in a precise manner. If the implant is offset from its intended position it may cause an increased wear or load on the joint. For example, if the implant is tilted, this may result in an edge that projects above the cartilage surface and causes wear on the opposing cartilage in the joint. Another example is the case that the implant is placed in a too shallow position, which may result in a too high top of the implant that causes the joint to articulate in an uneven manner and increase the load on an opposing point of the joint. For the patient, also small misplacements or deviations from an ideal position may result in pain, longer time for convalescence or even a surgical operation being done in vain and making it more difficult to repair the damage in the joint. A large burden is therefore placed on the surgeon not to misplace or misfit the implant. There is therefore a need for tools that are designed to relieve and support the surgeon in the implant surgery.

The design of the implant and the surgical tools, in other words, the design of the surgical kit, is crucial for the outcome of the implant's life-time in a joint. Also, the parameters for designing are of uttermost importance for the result in these operations. Small differences in the design can make a huge difference in fit and life-time of an implant in the body, convalescence time for the patient, economic values due or surgery time, and success of operations. Also, the number of successful operations will increase, and the working conditions for the surgeon will be improved, if the designing parameters are selected right.

There is a need for a design method for tools for use during repair of a cartilage damage which is more user friendly for the surgeon than the tools known from prior art. There is a need for tools which allow for small surgical cuts, and also a design method which allows for producing tools which are adapted to avoid problems with small misplacements or deviations from an ideal position, e.g. that the implant is tilted during insertion causing pain and longer convalescence time for the patient, and which are still stable and easy to use for the surgeon allowing for precise insertion of implants in a joint.

### Prior art

A prior art document that describes the design of an orthopedic implants and corresponding tools is for example EP2389905 A1, which shows a design method for designing an individually designed surgical kit.

### Object of the Invention

The general object of the invention is to solve the problem of designing an improved surgical kit for use during cartilage repair for replacing damaged cartilage and also an improved design method for designing a mandrel for hammering, pressing and/or pushing an implant into a recess made in a joint.

In aspects, the object of the invention is to design a mandrel which makes the surgical operation more accurate and safer, and which provides for less surgeon dependent operation procedures and faster recovery of the patients after surgery.

In certain aspects, the object of the invention is to design a mandrel for an implant which make the surgical operation safer and results in better fitting implants, less surgeon dependent operation procedures and faster recovery of the patients after surgery. It is a further object of the invention to provide a solution for making the surgical operation of rotationally positioning and inserting an implant in a recess safer and more accurate by providing a mandrel with at least one element and/or positioning mark which is adapted for rotationally positioning the mandrel in relation to at least one of an anatomic dependent direction, at least one element and/or positioning mark of the implant, and a mark to be made on side of a recess to be made at the determined implant position.

### Summary of the invention

A method for designing a mandrel according to the invention is defined in claim 1.

In embodiments, the method further comprises determining the position of the at least one element and/or positioning mark of the mandrel so that said at least one element and/or positioning mark is adapted for rotational positioning of the mandrel in relation to an element and/or a positioning mark of the implant. This enables a very exact positioning of the mandrel onto the implant to be implanted.

In embodiments, the method further comprises determining the position of the at least one element and/or positioning mark of the mandrel so that said at least one element and/or positioning mark is adapted for rotational positioning of the mandrel in relation to a mark on the side of the recess where the implant is to be inserted.

In embodiments, the method further comprises determining the position of the at least one element and/or positioning mark of the mandrel so that said at least one element and/or positioning mark is adapted for rotational positioning of the mandrel in relation to an anatomic dependent direction.

In embodiments, the method further comprises forming the contacting surface of the mandrel into a substantially circular shape, and designing the at least one element and/or positioning mark as a marking, such as e.g. a dot, or a groove in the circumference of the substantially circular shape.

In embodiments, the method further comprises forming the contacting surface of the mandrel from at least two substantially circular shapes, such that each of said substantially circular shapes is partly overlapping at least one other substantially circular shape, and designing the at least one element and/or positioning mark as a marking, such as e.g. a dot, or a groove in the circumference of at least one of said at least two substantially circular shapes.

In embodiments, the method further comprises designing at least a portion of the contacting surface of the mandrel to have an inverted surface, or essentially inverted surface, to the articulate surface of the implant to be inserted in the recess using the mandrel.

In embodiments, the method further comprises receiving image data representing a three-dimensional image of a joint; identifying cartilage damage in the image data; determining the position for the implant to be used for cartilage repair; simulating a healthy surface of the area of damaged cartilage; and designing the articulate surface of the implant to match the simulated healthy surface.

In embodiments, the method further comprises simulating said healthy surface based on image data representing a three-dimensional image of a joint and the curvature of the cartilage immediately surrounding the area of damaged cartilage.

In embodiments, the method further comprises designing the mandrel with a grip portion that has a smaller diameter close to the contacting surface than at the middle of the grip portion. It is an advantage to design the mandrel so that the point of gravity lies in the hand of the surgeon using the mandrel rather than close to the contacting surface. Further, if the grip portion has a larger diameter towards the middle, it may lie better in the hand of the surgeon.

A mandrel according to the invention is defined in claim 11.

In embodiments, the at least one element and/or positioning mark of the mandrel is adapted for rotational positioning of the mandrel in relation to at least one of at least one element and/or positioning mark on the surface of the implant, a mark on the side of the recess where the implant is to be inserted, and an anatomic dependent direction.

In embodiments, the contacting surface of the mandrel has a substantially circular shape, and the at least one element and/or positioning mark is a marking, such as e.g. a dot, or a groove in the circumference of the substantially circular shape.

In embodiments, the contacting surface of the mandrel is formed from at least two substantially circular shapes, such that each of said substantially circular shapes is partly overlapping at least one other substantially circular shape, and the at least one element and/or positioning mark is a marking, such as e.g. a dot, or a groove in the circumference of at least one of said at least two substantially circular shapes.

In embodiments, at least a portion of the contacting surface of the mandrel has an inverted surface, or essentially inverted surface, to the articulate surface of the implant to be inserted into the recess using the mandrel.

In embodiments, the contacting surface of the mandrel has a cross-sectional profile that is designed to correspond to the cross-sectional profile of the implant to be inserted into the recess using the mandrel, with a tolerance preventing the mandrel from coming into contact with the surrounding cartilage during insertion of the implant into the recess.

In embodiments, the mandrel further comprises a grip portion that has a smaller diameter close to the contacting surface than at the middle of the grip portion. It is an advantage to design the mandrel so that the point of gravity lies in the hand of the surgeon using the mandrel rather than close to the contacting surface. Further, if the grip portion has a larger diameter towards the middle, it may lie better in the hand of the surgeon.

The technology disclosed relates to a design method for designing a mandrel for hammering, pressing and/or pushing an implant into position in a recess made in a joint and firmly attach the implant to the bone of a patient, comprising determining a contacting surface of the mandrel to be in contact with the articulate surface of the implant during the insertion of the implant to fit the articulate surface of the implant in that the contacting surface of the mandrel has a corresponding cross-sectional profile.

The implant which is hammered/pressed into a recess using the mandrel is an individually customized implant designed with an articulate surface having a shape and curvature which is simulating a healthy surface of the area of damaged cartilage at the determined implant position where the implant is to be inserted. The contacting surface of the mandrel to be in contact with the articulate surface of the implant (which is to be inserted into a joint using the mandrel) is then designed to have an inverted surface curvature to the surface curvature of the articulate surface of the implant.

A mandrel may according to the invention be any type of tool that may be used for tapping, hammering, pressing and/or pushing an implant into position into a recess made in a joint of a patient.

### Brief description of the figures

The invention will be further explained with reference to the accompanying drawings which are exemplified embodiments according to the invention and not limiting to the scope of the invention.
Figure 1 shows an example of a surgical kit designed according to one or more embodiments of the invention.
Figures 2-4 show different embodiments of a mandrel according to one or more embodiments of the invention.
Figures 5a-b show medical implants which may be used with the invention.
Figure 6 shows a guide tool which may be used with one or more embodiments of the invention.
Figure 7 shows a guide tool comprising an implant dummy placed inside the guide channel of the guide tool.
Figure 8 shows the use of a mandrel for for hammering, pressing and/or pushing an implant into position in a recess made in a joint, according to one or more embodiments of the invention.

### Detailed description of the invention

The technology disclosed relates to a design method for designing a mandrel for hammering, pressing and/or pushing an implant into position in a recess made in a joint and firmly attaching the implant to the bone of a patient, comprising determining a surface of the mandrel to be in contact with the articulate surface of the implant during the insertion of the implant to fit the articulate surface of the implant in that the contacting surface of the mandrel has a corresponding cross-sectional profile.

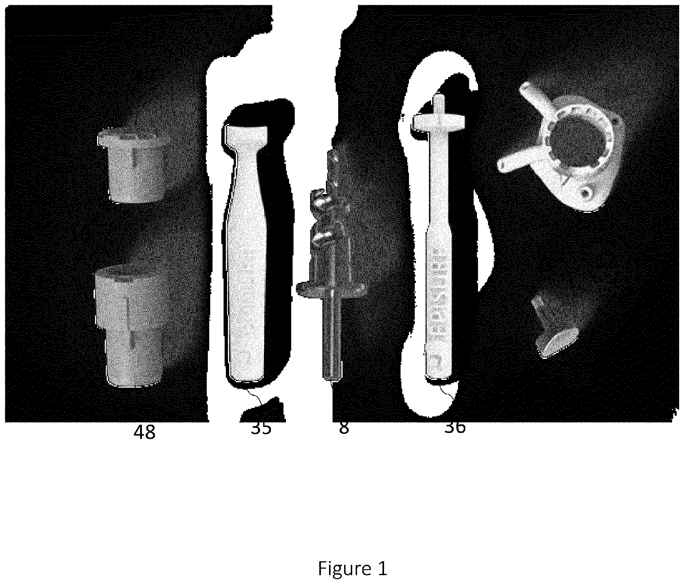

Figure 1 shows an example of a surgical kit. This particular example of a surgical kit is especially adapted for cartilage replacement at the femur of a knee joint. The invention may however be applied for cartilage replacement in an articulate surface in any other joint in the body, e.g. elbow, ankle, finger, hip, toe and shoulder. The surgical kit may e.g. comprise a height adjustment device 16, a hammer tool/mandrel 35, a drill-bit or reamer-bit 8, an implant dummy 36, a guide tool 12, and an implant 10. A surgical kit may additionally comprise e.g. a cartilage cutting tool or cartilage cutter, a cartilage cut drill, a punch, a reamer guide, and/or a drill guide.

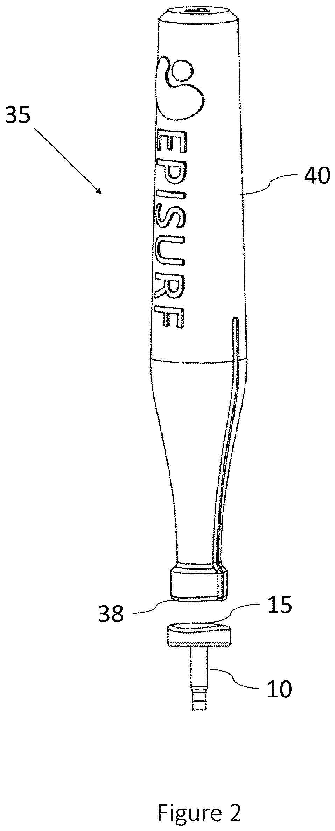

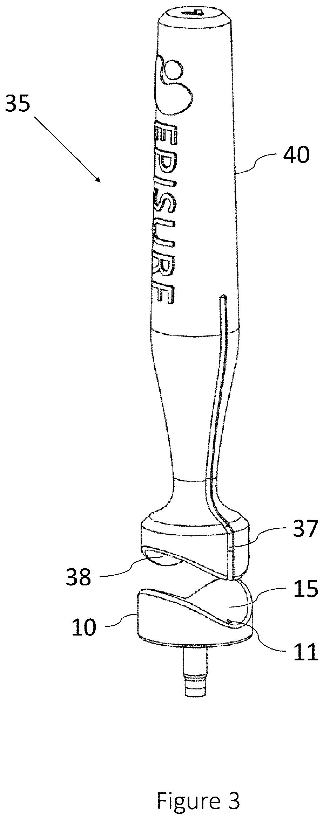

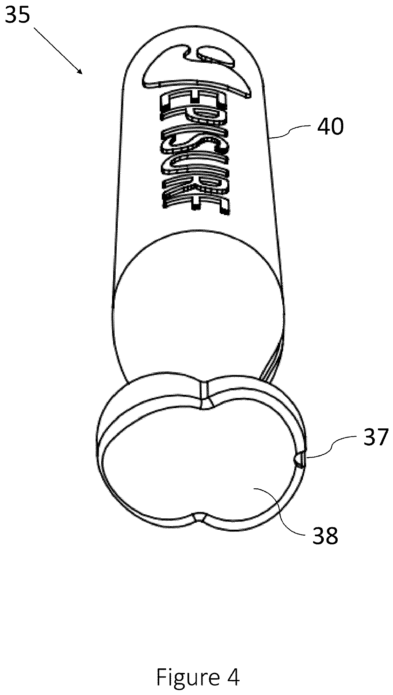

Figures 2-4 show different embodiments of a mandrel 35 according to the invention having a grip portion 40 and a contacting surface 38 designed to be in contact with an articulate surface 15 of an implant 10 during insertion of the implant 10 by hammering, pressing and/or pushing the implant 10 into position in a recess made in a joint. The contacting surface 38 of the mandrel 35 is designed to have an inverted surface to the articulate surface 15 of the implant 10 to be implanted. The mandrel 35 preferably comprises an element and/or a positioning mark 37 for rotational positioning of the mandrel 35 in the recess. The element and/or positioning mark 37 is preferably arranged to allow the simultaneous rotational positioning of the implant 10 in the recess. The contacting surface 38 of the mandrel 35 is preferably the same size, or slightly smaller than, the surface 15 of the implant 10 to be implanted. If the contacting surface 38 of the mandrel 35 has a cross-sectional profile that has a tolerance with respect to the cross-sectional profile of the implant 10 to be inserted into the recess using the mandrel 35, this may prevent the mandrel 35 from coming into contact with the surrounding cartilage during insertion of the implant 10 into the recess. The diameter of the surface 38 may be larger or smaller than the diameter of the grip portion 40 of the mandrel 35. The mandrel 35 shown in figure 4 is intended for use with an implant 10 formed from two substantially circular shapes such that each of said substantially circular shapes is partly overlapping the other substantially circular shape.

The mandrel 35 shown in figures 2-4 further comprises a grip portion 40, which is intended to provide a good grip for the surgeon using the mandrel 35. The grip portion 40 is preferably shaped to be thicker in the middle, and have a smaller diameter close to the contacting surface 38 than at the middle of the grip portion 40. It is an advantage to design the mandrel so that the point of gravity lies in the hand of the surgeon using the mandrel 35 rather than close to the contacting surface 38. Further, if the grip portion 40 has a larger diameter towards the middle, it may lie better in the hand of the surgeon.

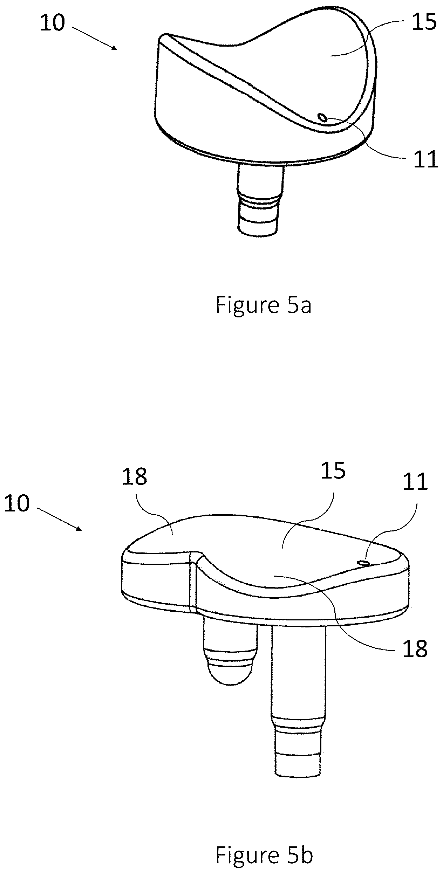

Figure 5a shows an example of a medical implant 10 provided with a positioning mark 11. The plate shaped implant body has an articulate surface (first surface) 15 configured to face the articulating part of the joint and a bone contact surface (second surface) configured to face bone structure in the joint. The plate shaped implant body has a cross-section that substantially corresponds to the area of the damaged cartilage and the articulate surface 15 has a curvature that substantially corresponds to the curvature of a healthy articulate surface at the site of diseased cartilage. The extending post extends from the bone contact surface. Since the implant 10 of the inventive concept is custom made for a specific patient, figure 5a is an exemplifying schematic picture displaying an embodiment of the implant 10. Between the articulate surface 15 and the bone contact surface there is a cartilage contacting surface.

The implant is specially designed, depending on the knees appearance and the shape of the damage and in order to resemble the body's own parts, having a surface which preferably corresponds to a three dimensional (3D) image of a simulated healthy cartilage surface. The implant will be tailor-made to fit each patient's damaged part of the joint.

The implant body is substantially plate shaped, meaning that the shortest distance crossing the surface 15 of the implant body is substantially larger, e.g. at least 1.5 times larger than the thickness of the implant body. By substantially plate shaped is meant that the implant body may be substantially flat or may have some curvature, preferably a 3D curvature of the articulate surface 15. The articulate surface 15 may for example have a curvature that corresponds to a simulated healthy cartilage reconstructed from an image taken e.g. with MRI or CT-scanning of the damaged cartilage surface of the joint. Once the implant 10 is placed in the joint there will be a surface with no parts of the implant pointing up from or down below the surrounding cartilage - the implant is incorporated to give a smooth surface.

The area and the shape of the implant surface 15 are individual depending on the size of cartilage damage and location of the cartilage damage. The area and shape of the implant can be decided by the surgeon himself or be chosen from predetermined shapes. For instance the cross-section of the implant body may have a circular or roughly circular, oval, triangular, square or irregular shape, preferably a shape without sharp edges.

In general, small implants are preferred since they have a smaller impact on the joint at the site of incision and are also more easily implanted using arthroscopy or smaller open surgical procedures. The primary factor for determining the size of the implant is however the nature of the lesion to be repaired.

The implant replaces an area of damaged cartilage in an articulate surface of a joint. Before the implant is placed in the desired position, the damaged cartilage is removed and also a part of the bone beneath, i.e. a recess fitting the implant is made in the bone. The recess can e.g. be drilled in the bone to fit the implant structure. The extending post or rod-part of the implant 10 is used for securing the implant 10 in the drilled hole of the bone. The length of the extending post, extending from the implant head, is adjusted to a length needed to secure the implant 10 in the bone. The extending post is intended to give a primary fixation of the implant 10. It provides mechanical attachment of the implant 10 to the bone in immediate connection with the surgical operation.

Figure 5b shows another example of a medical implant 10, where the implant 10 comprises two substantially circular shapes 18, where one of the circular shapes 18 is provided with a positioning mark 11 on its articulate surface 15.

The positioning mark 11 on the articulate surface 15, i.e. the top surface 15 facing the articulating part of the joint, of the medical implant 10 illustrated in figures 5a-b is designed to be used for determining the orientation in which the implant 10 is to be placed in a recess made in a damaged articulate surface of a joint.

The positioning mark 11 may be designed so that the direction of the positioning mark 11 is determining the placement orientation of the implant 10 in a recess, in that the placement orientation of the positioning mark 11 is also to be indicated by a mark made on the side of a recess made in the articulate surface of a joint in which the implant 10 is to be inserted, thereby providing for a correct or more accurate orientation of the implant when inserted in the recess made in a damaged articulate surface of a joint.

The positioning mark 11 on the articulate surface 15, i.e. the top surface 15 facing the articulating part of the joint, of the implant 10 may be designed so that the direction of the positioning mark 11 is designed to be pointing in an anatomic dependent direction in relation to a recess made in the articulate surface of a joint in which the implant 10 is to be inserted, thereby providing for a correct or more accurate orientation of the implant 10 when inserted in the recess made in a damaged articulate surface of a joint.

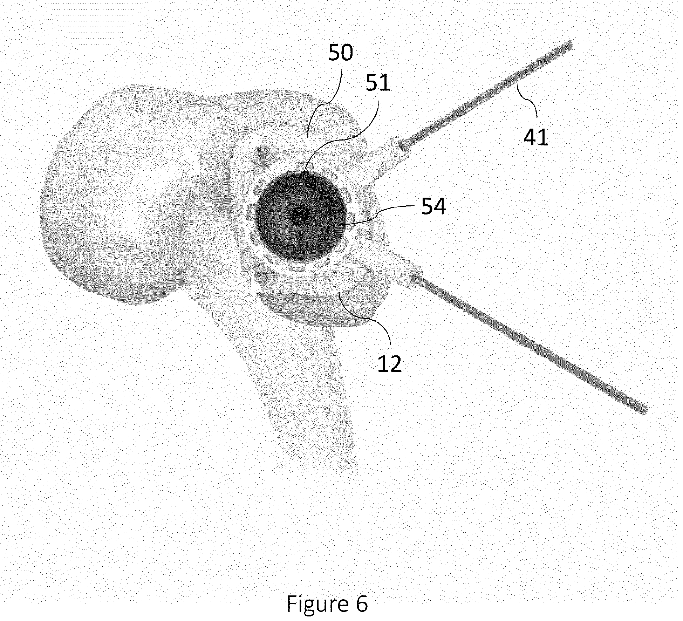

Figure 6 shows a guide tool 12 which may be used with embodiments of the invention. The guide tool 12 preferably has a cartilage contact surface that has a shape and contour that is designed to correspond to and to fit the contour of the cartilage or the subchondral bone in the joint in a predetermined area surrounding the site of diseased cartilage. The guide tool 12 aids with exact precision removal of a volume of cartilage and subchondral bone, and preferably has a guide channel 54. The guide tool 12 preferably comprises a positioning mark 50, comprised in the structure of the guide tool 12, wherein the positioning mark 50 is aligned with the centre of the guide channel 54 in a joint axis direction. The guide tool 12 may also comprise an indentation 51 that enables marking of the cartilage surface in the position of the positioning mark 50.

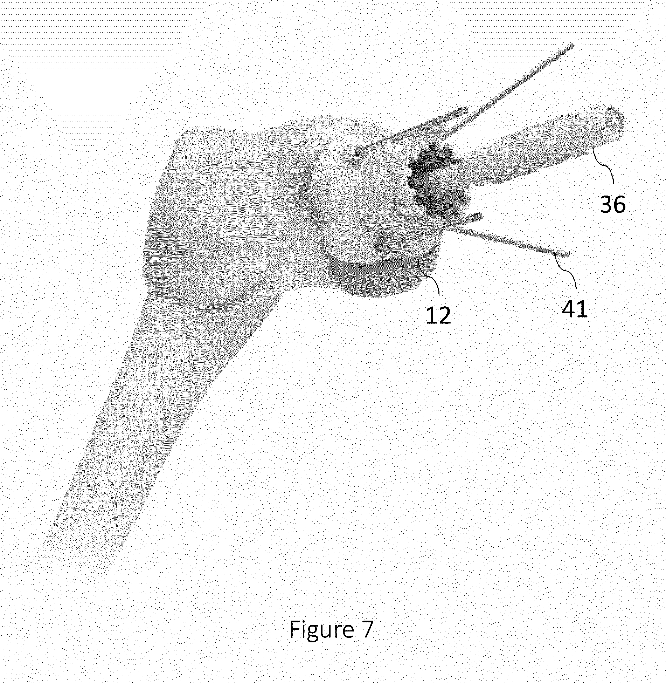

Figure 7 shows a guide tool 12 comprising an implant dummy 36 placed inside the guide channel 54 of the guide tool 12. The guide tool 12 may be placed in the joint using pins 41 for stabilization and fastening. The guide channel 54 is used for stabilizing tools that are to be inserted into the guide channel 54, such as e.g. a drill bit 8 and/or an implant dummy 36. The guide channel 54 therefore preferably has an inner cross-sectional profile that is designed to correspond to the cross-section of the drill bit 8 and/or the implant dummy 36. In other words, the drill bit 8 and/or the implant dummy 36 fits the guide channel 54, with a slight tolerance to allow a sliding movement of the drill bit 8 and/or the implant dummy 36 in the guide channel 54.

The guide channel 54 has an opening on the cartilage contact surface, arranged to be placed in a position corresponding to the site of the diseased cartilage in a joint. The height of the guide channel 54 must be sufficiently long to give support to the tools used inside the guide channel 54. The height of the guide channel 54 may e.g. be between 1 and 10 cm, preferably 3-10 cm, and always sufficiently high to ensure stabilization of the tools that are to be inserted into the guide channel 54. In one example, the top of the guide channel 54 is designed to project above the tissue surrounding the surgery cut when the guide tool is placed on the cartilage in a joint during surgery.

The guide tool 12 is easy to place due to the precise fit of the cartilage contact surface on the cartilage surface. The size and shape of cartilage contact surface of the guide tool 12 is determined depending on the size and shape of the damaged cartilage and thus on the cross section of the implant body 10, and also depending on the position of the cartilage area in a joint. The size, shape or spread of the cartilage contact surface of the guide tool 12 is a consideration between the following aspects; minimize surgery lesion, maximize stability for guide tool 12, anatomic limitations on the site of the injury. Not all cartilage surfaces in a joint can be used for placement of the guide tool 12. A large spread of the cartilage contact surface is preferable to get good stability of the guide tool 12, however, a large surface area of the surface may also lead to a large surgical intervention which is undesired.

Thus the size of the guide tool 12 is determined by a balance between the desire to achieve good positioning stability and small surgical operations. Also, the cartilage contact surface need not have a continuous, regular shape, but may have an irregular shape, as long as it gives adequate support and stable positioning of the guide tool 12. The cartilage contact surface may also consist of three separated points.

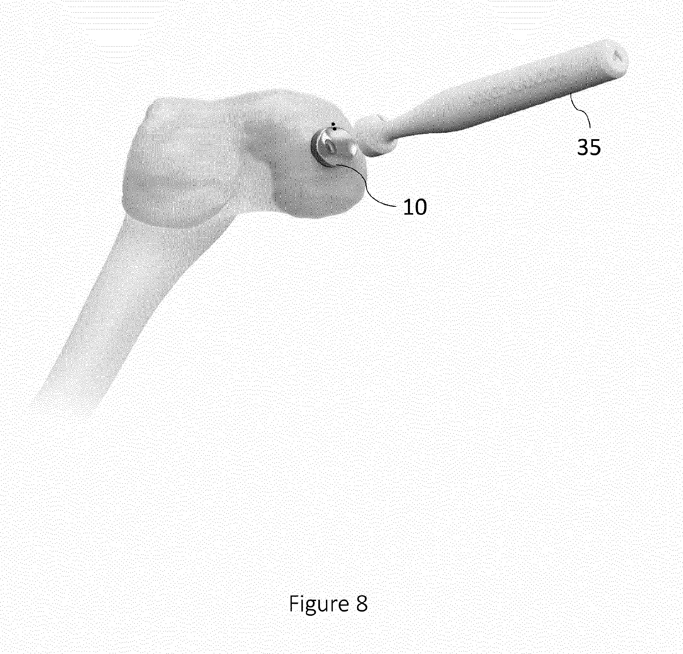

Figure 8 shows the use of a mandrel 35 for for hammering, pressing and/or pushing an implant 10 into position in a recess made in a joint, according to one or more embodiments of the invention. Figure 8 also shows a positioning mark indicated on the cartilage surface. The positioning mark is preferably added to the cartilage surface when the guide tool 12 is placed in the joint, e.g. by inserting a marking pen into the indentation 51 in the guide tool 12. The implant 10 is placed in the recess, rotated so that the positioning mark 11 of the implant 10 corresponds to the positioning mark indicated on the cartilage surface. The element and/or positioning mark 37 on the mandrel 35 is then preferably aligned with the positioning mark 11 on the implant 10 and the positioning mark indicated on the cartilage surface.

The element and/or a positioning mark 37 on the mandrel 35 is preferably designed to allow the surgeon to view the the positioning mark 11 of the implant 10 when the mandrel 35 is used for hammering, pressing and/or pushing the implant 10 into position in a recess.

The method for inserting an implant 10 into a recess by use of a mandrel 35 further comprises: providing a guide tool 12 comprising a positioning feature/mark 50; and making, by one of a surgeon and a mechanical arm such as a robot arm, a mark on the side of a recess made in an articulate surface of the joint in the direction of the positioning mark of said guide tool 12, thereby determining the future placement orientation of the implant 10. The surgical kit may also comprise a height adjustment device 16, e.g. comprising a male part 47 and a female receiving part 48 which when used together allows for stepwise adjustment of drill depth.

The male part 47 is in the outermost position in a zero-mode and may from there be adjusted inwards allowing the surgeon to for example make stepwise deeper drill holes. When the height adjustment device 16 is in starting mode or outermost zero-mode, the position marking of the guide tool 12 and the positioning marking of the height adjustment device 16 are aligned.

Thus, by being able to adjust the length of the guide channel 54, the surgeon is also able to adjust the depth of drilling and cutting into the bone. The length of the guide channel 54 may be varied since the guide tool 12 and the height adjustment device 16 are able to move in relation to one another. Further, the male part 47 and the female receiving part 48 of the height adjustment device 16 may be arranged such that the length of the guide channel 54 may be varied at certain stepwise intervals, e.g. at 200 µm or at 100-300 µm intervals or steps, or any other desired interval. For example, the height might be adjusted between for example 0.2-3 mm, in one or several steps. This may for instance be achieved by arranging the male part 47 inside the female receiving part 48 of the height adjustment device 16 such that the male part 47 insert tool to have a cross-sectional profile that corresponds to the cross-sectional profile of the guide channel of the female part 48 with a tolerance enabling the insert tool to slide within the guide channel of the female part 48.

The insert tool may e.g. be placed in a starting position where both the positioning mark of the insert tool is aligned with the positioning mark 50 of the guide tool 12.

According to the invention, the mandrel 35 has a contacting surface 38 that is designed to fit the articulate surface 15 of an implant 10. In certain embodiments, the contacting surface 38 of the mandrel 35 according to the technology disclosed may have a cross-sectional profile that is designed to correspond to the cross-sectional profile of the implant 10, with a tolerance preventing the mandrel 35 from coming into contact with the surrounding cartilage during insertion of the implant 10 into the recess. The contacting surface 38 of the mandrel 35 is thus preferably slightly smaller than the articulate surface 15 of the implant 10 all around the circumference.

The mandrel 35 according to the invention has a contacting surface 38 that is designed to fit the articulate surface 15 of the implant 10, i.e. it has a corresponding cross-sectional profile and preferably also a corresponding, although inverted, curvature. The mandrel 35 is preferably used to hammer and/or press the implant 10 in place.

The mandrel or hammer tool 35 may also be accompanied by a hammer tool adapter, for facilitating the use of the mandrel 35 and minimizing the absorption of the shock caused by the mandrel 35 and/or minimizing the risk of scratching the surface of the implant while hammering/pressing/pushing. Such a hammer tool adapter may be made from a soft material that is gentle to the implant surface, e.g. a rubber or plastic material.

The implant 10 is an individually customized implant designed with an articulate surface 15 having a shape and curvature which is simulating a healthy surface of the area of damaged cartilage at the determined implant position where the implant 10 is to be inserted, and at least a portion of the contacting surface 38 of the mandrel 35 to be in contact with the articulate surface 15 of the implant 10 is designed to have a corresponding cross-sectional profile.

The present invention may relate to different alternatives described in any combination, providing they are within the scope of the appended claims.

## Claims

1. A method for designing a mandrel (35) for hammering, pressing and/or pushing an implant (10) into position in a recess made in a joint, the mandrel (35) comprising a contacting surface (38) adapted to be in contact with an articulate surface (15) of the implant (10) to be inserted, the method comprising providing the mandrel (35) with at least one element and/or positioning mark (37) adapted to be used for determining the rotational orientation of the mandrel (35) in relation to the implant (10), in order to enable the mandrel (35) to be rotationally positioned so that the at least one element and/or positioning mark (37) on the mandrel (35) is aligned with a positioning mark (11) of the implant (10), and/or a mark on the side of the recess where the implant (10) is to be inserted, **characterised by** the method further comprising designing the contacting surface (38) of the mandrel (35) to be an inverted surface to a healthy surface of an area of damaged cartilage where the implant (10) is to be inserted, where the healthy surface is simulated based on image data representing a three-dimensional image of the joint and the curvature of the cartilage immediately surrounding the area where the implant (10) is to be inserted.

2. The design method according to claim 1, further comprising determining the position of the at least one element and/or positioning mark (37) of the mandrel (35) so that said at least one element and/or positioning mark (37) is adapted for rotational positioning of the mandrel (35) in relation to an element and/or a positioning mark (11) of the implant (10).

3. The design method according to claim 1 or 2, further comprising determining the position of the at least one element and/or positioning mark (37) of the mandrel (35) so that said at least one element and/or positioning mark (37) is adapted for rotational positioning of the mandrel (35) in relation to a mark on the side of the recess where the implant (10) is to be inserted.

4. The design method according to any of claims 1-3, further comprising determining the position of the at least one element and/or positioning mark (37) of the mandrel (35) so that said at least one element and/or positioning mark (37) is adapted for rotational positioning of the mandrel (35) in relation to an anatomic dependent direction.

5. The design method according to any of claims 1-4, further comprising forming the contacting surface (38) of the mandrel (35) into a substantially circular shape, and designing the at least one element and/or positioning mark (37) as a marking, such as e.g. a dot, or a groove in the circumference of the substantially circular shape.

6. The design method according to any of claims 1-4, further comprising forming the contacting surface (38) of the mandrel (35) from at least two substantially circular shapes, such that each of said substantially circular shapes is partly overlapping at least one other substantially circular shape, and designing the at least one element and/or positioning mark (37) as a marking, such as e.g. a dot, or a groove in the circumference of at least one of said at least two substantially circular shapes.

7. The design method according to any of claims 1-6, further comprising designing at least a portion of the contacting surface (38) of the mandrel (35) to have an inverted surface, or essentially inverted surface, to the articulate surface (15) of the implant (10) to be inserted in the recess using the mandrel (35).

8. The design method according to claim 7, further comprising:
receiving image data representing a three-dimensional image of a joint;
identifying cartilage damage in the image data;
determining the position for the implant (10) to be used for cartilage repair;
simulating a healthy surface of the area of damaged cartilage; and
designing the articulate surface (15) of the implant (10) to match the simulated healthy surface.

9. The design method according to claim 8, further comprising simulating said healthy surface based on image data representing a three-dimensional image of a joint and the curvature of the cartilage immediately surrounding the area of damaged cartilage.

10. The design method according to any of claims 1-9, further comprising designing the mandrel (35) with a grip portion (40) that has a smaller diameter close to the contacting surface (38) than at the middle of the grip portion (40).

11. A mandrel (35) configured for hammering, pressing and/or pushing an individually customized implant (10) designed with an articulate surface (15) having a shape and curvature which is simulating a healthy surface of an area of damaged cartilage of a joint into position in a recess made in said area of damaged cartilage of a joint, wherein said mandrel (35) comprises a contacting surface (38) adapted to fit said articulate surface (15) of the implant (10) to be inserted, and is provided with at least one element and/or positioning mark (37) adapted to be used for determining the rotational orientation of the mandrel (35) in relation to the implant (10), in order to enable the mandrel (35) to be rotationally positioned so that the at least one element and/or positioning mark (37) on the mandrel (35) is aligned with a positioning mark (11) of the implant (10), and/or a mark on the side of the recess where the implant (10) is to be inserted.

12. The mandrel (35) according to claim 11, wherein said at least one element and/or positioning mark (37) of the mandrel (35) is adapted for rotational positioning of the mandrel (35) in relation to at least one of at least one element and/or positioning mark (11) on the surface of the implant (10), a mark on the side of the recess where the implant (10) is to be inserted, and an anatomic dependent direction.

13. The mandrel (35) according to claim 11 or 12, wherein the contacting surface (38) of the mandrel (35) has a substantially circular shape, and the at least one element and/or positioning mark (37) is a marking, such as e.g. a dot, or a groove in the circumference of the substantially circular shape.

14. The mandrel (35) according to claim 11 or 12, wherein the contacting surface (38) of the mandrel (35) is formed from at least two substantially circular shapes, such that each of said substantially circular shapes is partly overlapping at least one other substantially circular shape, and the at least one element and/or positioning mark (37) is a marking, such as e.g. a dot, or a groove in the circumference of at least one of said at least two substantially circular shapes.

15. The mandrel (35) according to any of claims 11-14, wherein at least a portion of the contacting surface (38) of the mandrel (35) has an inverted surface, or essentially inverted surface, to the articulate surface (15) of the implant (10) to be inserted into the recess using the mandrel (35).

16. The mandrel (35) according to any of claims 11-15, wherein the contacting surface (38) of the mandrel (35) has a cross-sectional profile that is designed to correspond to the cross-sectional profile of the implant (10) to be inserted into the recess using the mandrel (35), with a tolerance preventing the mandrel (35) from coming into contact with the surrounding cartilage during insertion of the implant (10) into the recess.

17. The mandrel (35) according to any of claims 11-16, further comprising a grip portion (40) that has a smaller diameter close to the contacting surface (38) than at the middle of the grip portion (40).

## Patentansprüche

1. Verfahren zum Entwerfen eines Dorns (35) zum Hämmern, Pressen und/oder Schieben eines Implantats (10) in eine in einem Gelenk ausgebildete Aussparung, wobei der Dorn (35) eine Kontaktoberfläche (38) umfasst, die dazu angepasst ist, mit einer Gelenkoberfläche (15) des einzusetzenden Implantats (10) in Kontakt zu stehen, wobei das Verfahren ein Bereitstellen des Dorns (35) mit mindestens einem Element und/oder einer Positionierungsmarkierung (37) umfasst, das/die dazu angepasst ist, zum Bestimmen der Drehausrichtung des Dorns (35) in Bezug auf das Implantat (10) verwendet zu werden, um zu ermöglichen, dass der Dorn (35) drehbar positioniert wird, so dass das mindestens eine Element und/oder die mindestens eine Positionierungsmarkierung (37) auf dem Dorn (35) mit einer Positionierungsmarkierung (11) des Implantats (10) und/oder einer Markierung auf der Seite der Aussparung, wo das Implantat (10) eingesetzt werden soll, ausgerichtet ist, **dadurch gekennzeichnet, dass** das Verfahren weiter ein Entwerfen der Kontaktoberfläche (38) des Dorns (35) umfasst, die eine invertierte Oberfläche zu einer gesunden Oberfläche eines Bereichs eines beschädigten Knorpels sein soll, in den das Implantat (10) eingesetzt werden soll, wobei die gesunde Oberfläche auf der Grundlage von Bilddaten simuliert wird, die ein dreidimensionales Bild des Gelenks und der Krümmung des Knorpels darstellen, der den Bereich unmittelbar umgibt, in den das Implantat (10) eingesetzt werden soll.

2. Entwurfsverfahren nach Anspruch 1, weiter umfassend ein Bestimmen der Position des mindestens einen Elements und/oder der mindestens einen Positionierungsmarkierung (37) des Dorns (35), so dass das mindestens eine Element und/oder die mindestens eine Positionierungsmarkierung (37) für eine Drehpositionierung des Dorns (35) in Bezug auf ein Element und/oder eine Positionierungsmarkierung (11) des Implantats (10) angepasst ist.

3. Entwurfsverfahren nach Anspruch 1 oder 2, weiter umfassend ein Bestimmen der Position des mindestens einen Elements und/oder der mindestens einen Positionierungsmarkierung (37) des Dorns (35), so dass das mindestens eine Element und/oder die mindestens eine Positionierungsmarkierung (37) für eine Drehpositionierung des Dorns (35) in Bezug auf eine Markierung auf der Seite der Aussparung angepasst ist, in die das Implantat (10) eingesetzt werden soll.

4. Entwurfsverfahren nach einem der Ansprüche 1-3, weiter umfassend ein Bestimmen der Position des mindestens einen Elements und/oder der mindestens einen Positionierungsmarkierung (37) des Dorns (35), so dass das mindestens eine Element und/oder die mindestens eine Positionierungsmarkierung (37) für eine Drehpositionierung des Dorns (35) in Bezug auf eine anatomisch abhängige Richtung angepasst ist.

5. Entwurfsverfahren nach einem der Ansprüche 1-4, weiter umfassend ein Formen der Kontaktoberfläche (38) des Dorns (35) in eine im Wesentlichen kreisförmige Form, und ein Entwerfen des mindestens einen Elements und/oder der mindestens einen Positionierungsmarkierung (37) als eine Markierung, wie beispielsweise ein Punkt, oder eine Rille im Umfang der im Wesentlichen kreisförmigen Form.

6. Entwurfsverfahren nach einem der Ansprüche 1-4, weiter umfassend Formen der Kontaktoberfläche (38) des Dorns (35) aus mindestens zwei im Wesentlichen kreisförmigen Formen, so dass jede der im Wesentlichen kreisförmigen Formen zumindest eine andere im Wesentlichen kreisförmige Form teilweise überlappt, und Entwerfen des mindestens einen Elements und/oder der mindestens einen Positionierungsmarkierung (37) als eine Markierung, wie beispielsweise ein Punkt, oder eine Rille im Umfang mindestens einer der mindestens zwei im Wesentlichen kreisförmigen Formen.

7. Entwurfsverfahren nach einem der Ansprüche 1-6, weiter umfassend ein Entwerfen mindestens eines Abschnitts der Kontaktoberfläche (38) des Dorns (35), um eine invertierte Oberfläche, oder eine im Wesentlichen invertierte Oberfläche zur Gelenkoberfläche (15) des Implantats (10) aufweist, das unter Verwendung des Dorns (35) in die Aussparung eingesetzt werden soll.

8. Entwurfsverfahren nach Anspruch 7, weiter umfassend:
Empfangen von Bilddaten, die ein dreidimensionales Bild eines Gelenks darstellen;
Identifizieren von Knorpelschäden in den Bilddaten;
Bestimmen der Position für das zur Knorpelreparatur zu verwendende Implantat (10);
Simulieren einer gesunden Oberfläche des Bereichs eines beschädigten Knorpels; und
Entwerfen der Gelenkoberfläche (15) des Implantats (10), um der simulierten gesunden Oberfläche zu entsprechen.

9. Entwurfsverfahren nach Anspruch 8, weiter umfassend ein Simulieren der gesunden Oberfläche auf der Grundlage von Bilddaten, die ein dreidimensionales Bild eines Gelenks und der Krümmung des Knorpels darstellen, der den Bereich eines beschädigten Knorpels unmittelbar umgibt.

10. Entwurfsverfahren nach einem der Ansprüche 1-9, weiter umfassend ein Entwerfen des Dorns (35) mit einem Griffabschnitt (40), der in der Nähe der Kontaktoberfläche (38) einen kleineren Durchmesser aufweist als in der Mitte des Griffabschnitts (40).

11. Dorn (35), der zum Hämmern, Pressen und/oder Schieben eines individuell angepassten Implantats (10) konfiguriert ist, das mit einer Gelenkoberfläche (15) entworfen ist, die eine Form und eine Krümmung aufweist, die eine gesunde Oberfläche eines Bereichs eines beschädigten Knorpels eines Gelenks in eine Position in einer Aussparung in dem Bereich eines beschädigten Knorpels eines Gelenks simuliert, wobei der Dorn (35) eine Kontaktoberfläche (38) umfasst, die an die Gelenkoberfläche (15) des einzusetzenden Implantats (10) angepasst ist, und mit mindestens einem Element und/oder einer Positionierungsmarkierung (37) bereitgestellt ist, die dazu angepasst ist, zum Bestimmen der Drehausrichtung des Dorns (35) in Bezug auf das Implantat (10) verwendet zu werden, um zu ermöglichen, dass der Dorn (35) drehbar positioniert wird, so dass das mindestens eine Element und/oder die mindestens eine Positionierungsmarkierung (37) auf dem Dorn (35) mit einer Positionierungsmarkierung (11) des Implantats (10) und/oder einer Markierung auf der Seite der Aussparung, wo das Implantat (10) eingesetzt werden soll, ausgerichtet ist.

12. Dorn (35) nach Anspruch 11, wobei das mindestens eine Element und/oder die mindestens eine Positionierungsmarkierung (37) des Dorns (35) für eine Drehpositionierung des Dorns (35) in Bezug auf mindestens ein Element und/oder eine Positionierungsmarkierung (11) auf der Oberfläche des Implantats (10), eine Markierung auf der Seite der Aussparung, wo das Implantat (10) eingesetzt werden soll, und eine anatomisch abhängige Richtung angepasst ist.

13. Dorn (35) nach Anspruch 11 oder 12, wobei die Kontaktoberfläche (38) des Dorns (35) eine im Wesentlichen kreisförmige Form aufweist, und das mindestens eine Element und/oder die mindestens eine Positionierungsmarkierung (37) eine Markierung ist, wie beispielsweise ein Punkt, oder eine Rille im Umfang der im Wesentlichen kreisförmigen Form.

14. Dorn (35) nach Anspruch 11 oder 12, wobei die Kontaktoberfläche (38) des Dorns (35) aus mindestens zwei im Wesentlichen kreisförmigen Formen ausgebildet ist, so dass jede der im Wesentlichen kreisförmigen Formen zumindest eine andere im Wesentlichen kreisförmige Form teilweise überlappt, und das mindestens eine Element und/oder die mindestens eine Positionierungsmarkierung (37) eine Markierung ist, wie beispielsweise ein Punkt, oder eine Rille im Umfang zumindest einer der zumindest zwei im Wesentlichen kreisförmigen Formen.

15. Dorn (35) nach einem der Ansprüche 11-14, wobei zumindest ein Abschnitt der Kontaktoberfläche (38) des Dorns (35) eine invertierte Oberfläche oder eine im Wesentlichen invertierte Oberfläche zur Gelenkoberfläche (15) des Implantats (10) aufweist, das unter Verwendung des Dorns (35) in die Aussparung eingesetzt werden soll.

16. Dorn (35) nach einem der Ansprüche 11-15, wobei die Kontaktoberfläche (38) des Dorns (35) ein Querschnittsprofil aufweist, das so entworfen ist, dass es dem Querschnittsprofil des Implantats (10) entspricht, das unter Verwendung des Dorns (35) in die Aussparung eingesetzt werden soll, wobei eine Toleranz verhindert, dass der Dorn (35) beim Einsetzen des Implantats (10) in die Aussparung mit dem umgebenden Knorpel in Kontakt kommt.

17. Dorn (35) nach einem der Ansprüche 11-16, weiter umfassend einen Griffabschnitt (40), der in der Nähe der Kontaktfoberläche (38) einen kleineren Durchmesser aufweist als in der Mitte des Griffabschnitts (40).

## Revendications

1. Procédé de conception d'un mandrin (35) pour marteler, presser et/ou pousser un implant (10) en position dans un évidement réalisé dans une articulation, le mandrin (35) comprenant une surface (38) de contact conçue pour être en contact avec une surface (15) articulée de l'implant (10) devant être inséré, le procédé comprenant la fourniture au mandrin (35) d'au moins un élément et/ou repère (37) de positionnement conçu pour être utilisé pour la détermination de l'orientation de rotation du mandrin (35) par rapport à l'implant (10), afin de pouvoir positionner le mandrin (35) en rotation de sorte que l'au moins un élément et/ou repère (37) de positionnement sur le mandrin (35) soit aligné avec un repère (11) de positionnement de l'implant (10), et/ou un repère sur le côté de l'évidement où l'implant (10) doit être inséré, **caractérisé en ce que** le procédé comprend en outre la conception de la surface (38) de contact du mandrin (35) comme une surface inversée vers une surface saine d'une zone de cartilage lésé où l'implant (10) doit être inséré, où la surface saine est simulée sur la base de données d'image représentant une image tridimensionnelle de l'articulation et de la courbure du cartilage entourant immédiatement la zone où l'implant (10) doit être inséré.

2. Procédé de conception selon la revendication 1, comprenant en outre la détermination de la position de l'au moins un élément et/ou repère (37) de positionnement du mandrin (35) de sorte que ledit au moins un élément et/ou repère (37) de positionnement soit adapté pour le positionnement en rotation du mandrin (35) par rapport à un élément et/ou repère (11) de positionnement de l'implant (10).

3. Procédé de conception selon la revendication 1 ou la revendication 2, comprenant en outre la détermination de la position de l'au moins un élément et/ou repère (37) de positionnement du mandrin (35) de sorte que ledit au moins un élément et/ou repère (37) de positionnement soit adapté pour le positionnement en rotation du mandrin (35) par rapport à un repère sur le côté de l'évidement où l'implant (10) doit être inséré.

4. Procédé de conception selon l'une quelconque des revendications 1 à 3, comprenant en outre la détermination de la position de l'au moins un élément et/ou repère (37) de positionnement du mandrin (35) de sorte que ledit au moins un élément et/ou repère (37) de positionnement soit adapté pour le positionnement en rotation du mandrin (35) par rapport à une direction dépendant de l'anatomie.

5. Procédé de conception selon l'une quelconque des revendications 1 à 4, comprenant en outre la formation de la surface (38) de contact du mandrin (35) sous une forme sensiblement circulaire, et la conception de l'au moins un élément et/ou repère (37) de positionnement en tant que repérage, tel qu'un point ou une rainure dans la circonférence de la forme sensiblement circulaire.

6. Procédé de conception selon l'une quelconque des revendications 1 à 4, comprenant en outre la formation de la surface (38) de contact du mandrin (35) à partir d'au moins deux formes sensiblement circulaires, de sorte que chacune desdites formes sensiblement circulaires chevauche partiellement au moins une autre forme sensiblement circulaire, et la conception de l'au moins un élément et/ou repère (37) de positionnement en tant que marquage, tel qu'un point, par exemple, ou une rainure dans la circonférence de l'au moins une desdites au moins deux formes sensiblement circulaires.

7. Procédé de conception selon l'une quelconque des revendications 1 à 6, comprenant en outre la conception d'au moins une partie de la surface (38) de contact du mandrin (35) pour présenter une surface inversée, ou sensiblement inversée, à la surface (15) articulée de l'implant (10) devant être inséré dans l'évidement à l'aide du mandrin (35).

8. Procédé de conception selon la revendication 7, comprenant en outre :
la réception de données d'image représentant une image tridimensionnelle d'une articulation ;
l'identification de lésions du cartilage dans les données d'image ;
la détermination de la position de l'implant (10) devant être utilisé pour la réparation du cartilage ;
la simulation d'une surface saine de la zone de cartilage lésé ; et
la conception de la surface (15) articulaire de l'implant (10) pour qu'elle corresponde à la surface saine simulée.

9. Procédé de conception selon la revendication 8, comprenant en outre la simulation de ladite surface saine sur la base de données d'image représentant une image tridimensionnelle d'une articulation et de la courbure du cartilage entourant immédiatement la zone de cartilage lésé.

10. Procédé de conception selon l'une quelconque des revendications 1 à 9, comprenant en outre la conception du mandrin (35) avec une partie (40) de préhension présentant un diamètre plus petit à proximité de la surface (38) de contact qu'au milieu de la partie (40) de préhension.

11. Mandrin (35) configuré pour marteler, presser et/ou pousser un implant (10) personnalisé individuellement conçu avec une surface (15) articulée présentant une forme et une courbure qui simulent une surface saine d'une zone de cartilage lésé d'une articulation en position dans un évidement réalisé dans ladite zone de cartilage lésé d'une articulation, dans lequel ledit mandrin (35) comprend une surface (38) de contact adaptée à ladite surface (15) articulée de l'implant (10) devant être inséré, et est doté d'au moins un élément et/ou repère (37) de positionnement conçu pour être utilisé pour déterminer l'orientation de rotation du mandrin (35) par rapport à l'implant (10), afin de pouvoir positionner le mandrin (35) en rotation de sorte que l'au moins un élément et/ou repère (37) de positionnement sur le mandrin (35) soit aligné avec un repère (11) de positionnement de l'implant (10), et/ou un repère sur le côté de l'évidement où l'implant (10) doit être inséré.

12. Mandrin (35) selon la revendication 11, dans lequel ledit au moins un élément et/ou repère (37) de positionnement du mandrin (35) est conçu pour le positionnement en rotation du mandrin (35) par rapport à au moins l'un d'au moins un élément et/ou repère (11) de positionnement sur la surface de l'implant (10), un repère sur le côté de l'évidement où l'implant (10) doit être inséré, et une direction dépendant de l'anatomie.

13. Mandrin (35) selon la revendication 11 ou la revendication 12, dans lequel la surface (38) de contact du mandrin (35) présente une forme sensiblement circulaire, et l'au moins un élément et/ou repère (37) de positionnement est un marquage, tel qu'un point ou une rainure dans la circonférence de la forme sensiblement circulaire.

14. Mandrin (35) selon la revendication 11 ou la revendication 12, dans lequel la surface (38) de contact du mandrin (35) est formée d'au moins deux formes sensiblement circulaires, de sorte que chacune desdites formes sensiblement circulaires chevauche partiellement au moins une autre forme sensiblement circulaire, et que l'au moins un élément et/ou repère (37) de positionnement est un marquage, tel qu'un point ou une rainure dans la circonférence de l'au moins une desdites au moins deux formes sensiblement circulaires.

15. Mandrin (35) selon l'une quelconque des revendications 11 à 14, dans lequel au moins une partie de la surface (38) de contact du mandrin (35) présente une surface inversée, ou sensiblement inversée, par rapport à la surface (15) articulée de l'implant (10) devant être inséré dans l'évidement à l'aide du mandrin (35).

16. Mandrin (35) selon l'une quelconque des revendications 11 à 15, dans lequel la surface (38) de contact du mandrin (35) présente un profil en coupe qui est conçu pour correspondre au profil en coupe de l'implant (10) devant être inséré dans l'évidement à l'aide du mandrin (35), avec une tolérance empêchant le mandrin (35) d'entrer en contact avec le cartilage environnant lors de l'insertion de l'implant (10) dans l'évidement.

17. Mandrin (35) selon l'une quelconque des revendications 11 à 16, comprenant en outre une partie (40) de préhension qui présente un diamètre plus petit à proximité de la surface (38) de contact qu'au milieu de la partie (40) de préhension.
